# EUROPEAN PATENT SPECIFICATION

(11) **EP 3 089 676 B1**
(45) Date of publication and mention of the grant of the patent: **27.03.2019**
(21) Application number: 14877446.6
(22) Date of filing: 19.12.2014
(51) Int. Cl.: A61B 17/04, A61B 17/062

(54) **STITCHING APPARATUS AND METHODS**
NÄHVORRICHTUNG UND VERFAHREN
APPAREIL ET PROCÉDÉS DE RÉALISATION DE POINTS DE SUTURE

(30) Priority: 31.12.2013 US 201361922595 P
(43) Date of publication of application: 09.11.2016
(73) Proprietor: Children's Medical Center Corporation, Boston, MA 02115 (US)
(72) Inventor: LIU, Kaifeng, Boston, MA 02115 (US)
(74) Representative: Dehns
(86) International application number: PCT/US2014/071599
(87) International publication number: WO 2015/102954

(56) References cited:
- EP-B1- 0 778 004
- WO-A1-91/06247
- WO-A1-2011/025767
- WO-A1-2012/101999
- DE-A1- 19 807 743
- US-A- 5 149 329
- US-A1- 2003 105 474
- US-A1- 2007 118 153
- US-A1- 2007 118 153
- US-A1- 2009 318 958
- US-A1- 2009 318 958
- US-A1- 2013 074 849

## Description

### BACKGROUND

### (a) Technical Field

The present invention relates to a stitching apparatus for placing a stitch through a layer of material such as tissue. In particular aspects, the present invention relates to stitching apparatus and methods that pierce through a layer of material such as tissue, engages a thread, and pulls the thread through the layer of material.

### (b) Background Art

Stitches are often used in a myriad of industries to bind two pieces of material together. For example, the medical industry often uses stitches to suture layers of tissue together during surgical procedures, thereby allowing the layers of tissue to fuse while healing. A suture needle is typically used to force a suture thread through the separate layers of tissue to allow the suture thread to bind the tissue layers. Document US2013/074849 discloses a system for the securement of tissue comprising: a securement fiber comprising a distal end and a proximal end, the distal end further comprising a distal connector and the proximal end further comprising a proximal connector; a needle comprising a proximal end and a distal end, the distal end further comprising a tissue piercing surface and the proximal end comprising a connector, the connector being releasably engageable with the distal connector of the fiber; and a tissue passer comprising a proximal end and a distal end, the distal end releasably engageable with the distal and proximal connectors of the fiber. The connector can be manufactured in a magnetic material.

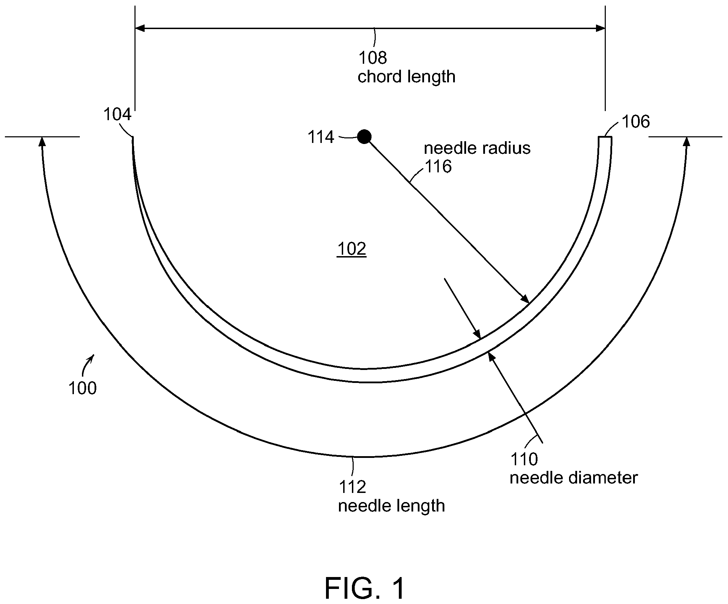

The present invention is disclosed in the appended set of claims. FIGS. 1-3B illustrate the configuration of a traditional suture needle used during medical procedures. As shown in FIG. 1, a typical suture needle 100 includes a needle body 102 having a needle point 104 on one end and a suture connection point 106 on the opposite end. Suture connection point 106 connects suture needle 100 to a suture thread and may be of an eyeless configuration. For example, suture connection point 106 may be swaged to an end of the suture thread. Generally, suture needles are classified based on their length, diameter and percentage of curvature relative to a center location. As shown, for example, suture needle 100 has a needle length 112, a diameter 110, and is a "half circle" type of suture needle since its length 112 extends along exactly half the perimeter of a circle having center 114 and radius 116. Other common types of suture needles (not shown) include "quarter circle," "three eights circle," and "five eights circle" suture needles. Typically, longer suture needles (e.g., three eights circle needles) are used for large or superficial wounds while shorter suture needles (e.g., half circle or five eights circle needles) are used for cases in which access or room for manipulation is limited, such as in small and deep spaces.

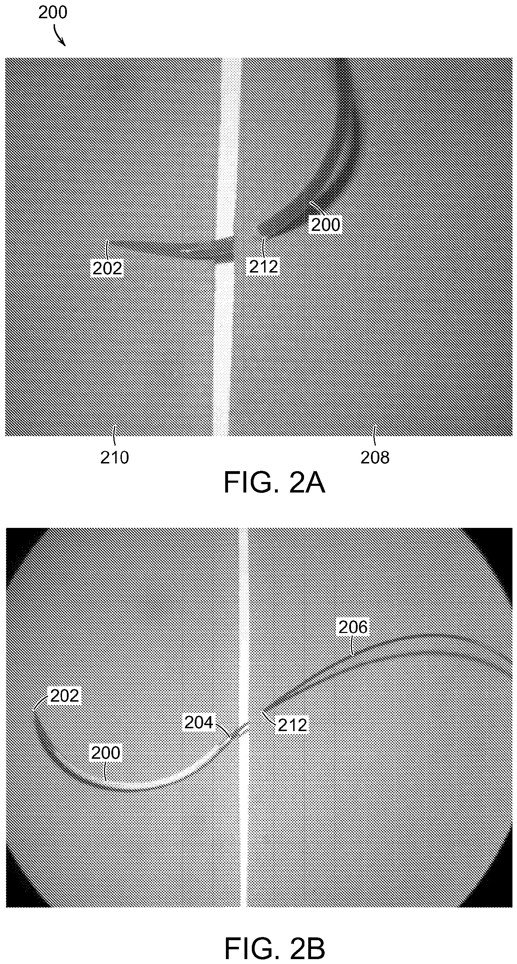

FIGS. 2A-2B depict the use of a suture needle 200 coupled with a suture thread 206 to first layer of material 208. As shown in FIG. 2A, needle point 202 of suture needle 200 is inserted through material 208 at puncture point 212 by applying an insertion force to suture needle 200, thereby penetrating material 208. Additional insertion force may then be used to drive the remainder of suture needle 200 through material 208, as shown in FIG. 2B. Suture thread 206, which is coupled to suture needle at connection point 204, is pulled along when the insertion force is applied to suture needle 200, thereby pulling suture thread 206 through puncture point 212 and to the opposite side of material 208. This process may then be repeated with a second layer of material 210 to bind materials 208, 210 together using suture thread 206.

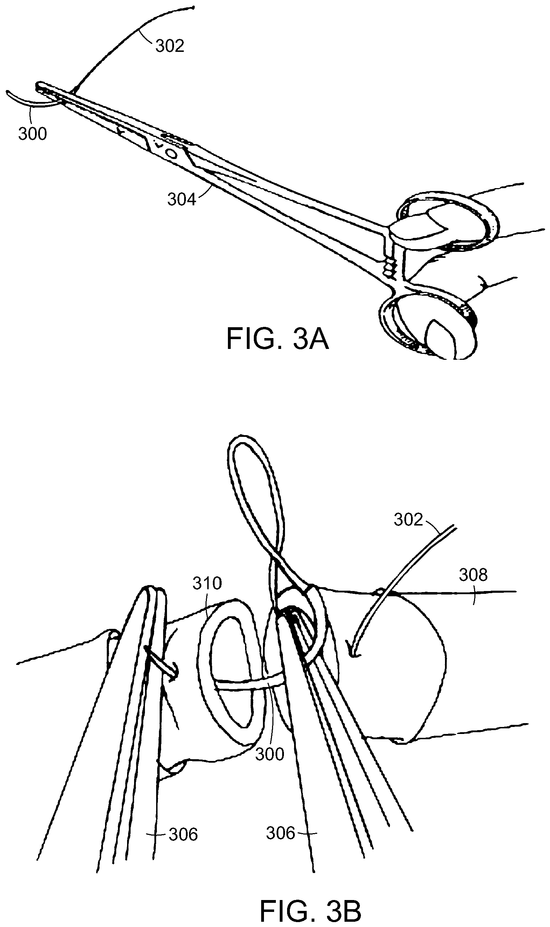

FIGS. 3A-3B illustrate the use of surgical tools that are commonly used in conjunction with a suture needle, when a medical procedure is performed. As shown in FIG. 3A, a suture needle 300 attached to a suture thread 302 may be gripped using a needle holder 304. A user operating needle holder 304 grasps one end of needle holder 304 to apply a compressive force to suture needle 300 that allows suture needle 300 to be manipulated using needle holder 304. In FIG. 3B, a vessel anastomosis procedure is illustrated in which suture thread 302 has been threaded through a first vessel wall 308 and is in the process of being threaded through a second vessel wall 310. By manipulating needle holder 304, the user is able to apply an insertion force to suture needle 300 to pierce vessel wall 310 and insert suture needle 300 through vessel wall 310. Typically, the user also uses a pair of tweezers 306 to grip the portion of suture needle 300 that has protrudes from vessel wall 310 after insertion of suture needle 300 through vessel wall 310. The user may then continue applying an insertion force to suture needle 300 by continuing to push suture needle 300 through vessel wall 310 and/or by pulling suture needle 300 through vessel wall 310 using tweezers 306. As a result of this process, suture thread 302 will eventually extend through both vessel walls 308, 310. The process is then repeated any number of times in an alternating manner between vessel walls 308, 310 to form stitches using suture thread 302, thereby binding vessel walls 308, 310 together.

In the stitching process described above, a traditional suture needle is required to have at least minimum length due to the use of a needle holder and tweezers. In other words, a traditional suture needle must be long enough to allow it to be gripped by both the needle holder and tweezers simultaneously, while still accounting for the thickness of the tissue. In addition, 3-4 times the amount of needle length space is needed in a traditional suture needle to prevent the needle from accidentally puncturing neighboring tissue. However, the size constraints on traditional suture needles also prevent them from accessing certain angles or surgical fields. For example, a traditional suture needle may be too large to access certain deep, small, narrow, or serpentine working spaces.

United States Patent Application Publication No. 2013/0074849 discloses a system and method for tissue securement that minimizes trauma. The system comprises a suspension fibre, a releasable needle and a linear suture passer.

United States Patent Application Publication No. 2007/0018153 discloses a suturing device that comprises an insertion puncture needle and several extraction devices.

### SUMMARY

We now provide systems and methods for passing a thread element through a material, particularly a suture through tissue of a subject that includes (a) piercing a needle element through tissue; (b) engaging a suture with the needle; and (c) pulling the suture through the tissue. The needle element releasably engages the suture element, the reliable engagement is magnetic.

We have found that the systems and methods of the invention can utilize a needle of particularly short dimensions (length) in comparison to traditional stitching tools. In the present methods, the needle element first pierces through tissue and then couples with and withdraws the suture element through the tissue puncture point already established by the first piercing of the needle. This can enable use of a needle element of comparatively small dimensions and suturing can be accomplished within confined spaces relative to prior approaches where a suture material and needle element are initially coupled together and that suture/needle assembly is advanced together for the first time through tissue. Additionally, by first establishing a puncture point in accordance with the present methods, the suture material can be advanced through the puncture point in the tissue with little resistance.

In preferred system, a needle element may comprise a thread extractor which engages an end of the thread element such as a suture. An end portion of the needle element comprises an enclosure that houses a thread extractor element and at least partially encases an end of the suture when the suture is engaged to the thread extractor element. The thread extractor comprises a magnet that engages a thread end portion by magnetically coupling the thread end portion to the thread extractor.

The stitching apparatus includes a handle portion configured to be gripped by a user and an insertion portion coupled to the handle portion that is configured to be inserted through a layer of material. The insertion portion includes a needle tip configured to pierce through the layer of material when an insertion force is applied to the needle tip. The insertion portion also includes an elongated base coupling the needle tip to the handle portion and configured to transfer the insertion force from the handle portion to the needle tip. The insertion portion further includes a thread extractor configured to engage a thread tip when the insertion portion has been inserted through the layer of material and further configured to transfer an extraction force from the handle portion to the thread tip to pull the thread tip through the layer of material.

The needle tip and elongated base define a hollow enclosure that houses the thread extractor and enshrouds the thread tip when the thread tip is engaged to the thread extractor. The elongated base and needle tip extend along an axis radial to the handle portion.

The thread extractor includes a magnet (e.g., a permanent magnet, an electromagnet, etc.) that engages the thread tip by magnetically coupling the thread tip to the thread extractor, one or more apertures that engage the thread tip, or one or more protrusions that engage the thread tip. In embodiments that include an electromagnet, the handle of the stitching apparatus may also include electronics configured to provide electrical power to the electromagnet. The electronics may further include a battery housed by the handle portion that supplies power to the electromagnet or an electronic switch configured to control the supply of power to the electromagnet when activated by the user.

In another preferred embodiment, stitching methods are provided that include transferring an insertion force from a handle to a needle tip and piercing through a layer of material using the needle tip to insert an insertion portion through the layer of material. The method also may include engaging a thread extractor of the insertion portion to a tip of a thread while the insertion portion is inserted through the layer of material. The method may further include transferring an extraction force from the handle to the tip of the thread. The method additionally may include pulling the thread through the layer of material by extracting the insertion portion from the layer of material.

In yet a further embodiment, a stitching apparatus is provided that includes a needle element which comprises a thread extractor which comprises a magnet that engages an end of the thread element by magnetically coupling the end to the thread extractor; a handle portion; an insertion portion that includes an elongated base extending radially from the handle portion at an angle and the elongated base being hollow with the thread extractor being located within the hollow interior, and a needle tip formed on the elongated base by tapering or beveling away from the handle portion, a thread, and means for transferring an insertion force from the handle portion to the needle tip in order to pierce through a layer of material using the needle tip to insert the insertion portion through the layer of material.

A variety of configurations of the disclosed stitching apparatus and methods will be suitable and preferred for many applications.

For instance, a magnet or other gripping force element may be molded or otherwise produced as a single unit with a needle element.

Alternatively, a magnet or other gripping force element may be separated (spaced and/or no physical attachment) to a needle element. In one preferred embodiment, the needle element suitably is within the magnetic field of the unattached magnet or within the attractive field of other gripping force element (such as a vacuum). In such systems, the needle element itself suitably would not need to be magnetized.

In other preferred embodiments, the needle element in whole or part may be magnetized. In such embodiments, with use of a hollow needle, the hollow needle lumen may serve as a path for entry of the thread element.

Needle elements of a variety of designs and configurations suitably can be employed. For instance, in addition to a hollow needle element, a needle element without a lumen suitably may be utilized. Both sharp and blunt-ended needles may be suitably employed.

Also, as mentioned, a thread element may be engaged to a needle element through one or more of a variety of attractive forces. Use of a vacuum also could be employed to engage the thread and needle elements. Mechanical and adhesive engagement of thread and needle elements also suitably can be utilized.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

### BRIEF DESCRIPTION OF THE DRAWINGS

The above and other features of the present invention will now be described in detail with reference to certain exemplary embodiments thereof illustrated the accompanying drawings which are given herein by way of illustration only, and thus are not limitative of the present invention, and wherein:
FIG. 1 is an illustration of a suture needle.
FIG. 2A is an image depicting a suture needle being inserted through a layer of material.
FIG. 2B is an image depicting the suture needle of FIG. 2A having been pulled entirely through the layer of material.
FIG. 3A is an illustration of a needle holder grasping a suture needle.
FIG. 3B is an illustration of a vessel anastomosis procedure being performed using the needle holder and suture needle of FIG. 3A with a pair of tweezers.
FIG. 4 is an image depicting a stitching apparatus, according to one exemplary embodiment.
FIG. 5 is an image depicting the insertion portion of a stitching apparatus in greater detail.
FIG. 6A is an image depicting an insertion force being applied to a stitching apparatus to puncture a layer of material.
FIG. 6B is an image showing a thread tip in close proximity to the stitching apparatus of FIG. 6A.
FIG. 6C is an image of the thread tip of FIG. 6B engaging a thread extractor of the stitching apparatus of FIGS. 6A-6B.
FIG. 6D is an image depicting an extraction force being applied to the stitching apparatus of FIGS. 6A-6C to pull the thread tip through the layer of material.
FIG. 7 is a schematic diagram of a stitching apparatus that uses an electromagnetic thread extractor.
FIG. 8 is a schematic diagram of a stitching apparatus having a curved insertion portion.
FIG. 9A is a cross-sectional view of the insertion portion of a stitching apparatus having locking protrusions.
FIG. 9B is a cross sectional view of a thread tip having apertures that engage the locking protrusions of FIG. 9A.

It should be understood that the appended drawings are not necessarily to scale, presenting a somewhat simplified representation of various preferred features illustrative of the basic principles of the invention. The specific design features of the present invention as disclosed herein, including, for example, specific dimensions, orientations, locations, and shapes will be determined in part by the particular intended application and use environment.

In the figures, reference numbers refer to the same or equivalent parts of the present invention throughout the several figures of the drawing.

### DETAILED DESCRIPTION

The present stitching apparatus and methods can provide numerous advantages. For instance, as discussed, the insertion portion of the stitching apparatus may be much shorter than that of traditional needles, such as suture needles. This can allow a user to be able to place stitches in traditionally inaccessible places. Additionally, the short insertion portion of the stitching apparatus can have less of a chance of accidentally puncturing neighboring tissue than traditional needles, when used in a surgical procedure.

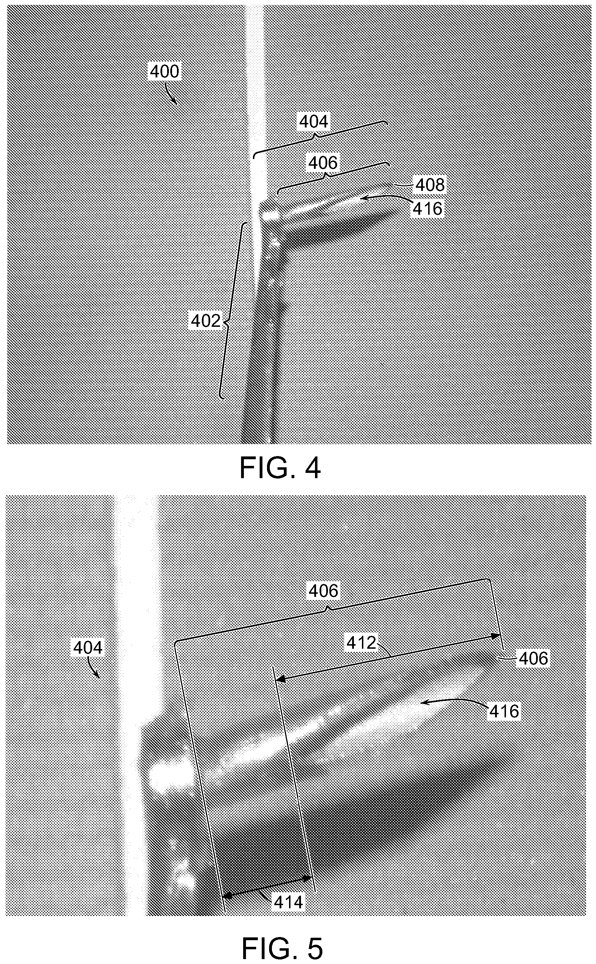

Referring now to FIG. 4 of the drawings, a preferred stitching apparatus 400 is shown, according to an exemplary embodiment. Stitching apparatus 400 includes a handle portion 402 that can be gripped by either hand of a user to manipulate stitching apparatus 400. Stitching apparatus 400 also includes an insertion portion 404 that can be at least partially inserted through a layer of material by manipulation of handle portion 402 by a user.

Handle portion 402 of stitching apparatus 400 may be of any size or shape, according to various embodiments. In some cases, handle portion 402 may be ergonomically shaped to fit one or both hands of a user. The width of handle portion 402 may also be uniform or may vary along the length of handle portion 402. For example, the width of handle portion 402 may be tapered closest to the junction between handle portion 402 and insertion portion 404, thereby minimizing the size of stitching apparatus 400 in the work area.

Insertion portion 404 of stitching apparatus 400 may be permanently affixed to handle portion 402 or may be detachable. In various embodiments, handle portion 402 and insertion portion 404 may be forged from a single piece of metal, insertion portion 404 may be welded or otherwise adhered to handle portion 402, or insertion portion 404 may engage an internal latching mechanism of handle portion 402 that prevents detachment of insertion portion 404 once latched to handle portion 402. In other embodiments, insertion portion 404 may releasably engage handle portion 402 via threading (e.g., insertion portion 404 may include threading and can be screwed into handle portion 402), a latching mechanism that can be actuated to release insertion portion 404 from handle portion 402 (e.g., via the push of a button, the movement of a switch, etc.), or any other mechanism that allows insertion portion 404 to be separated from handle portion 402.

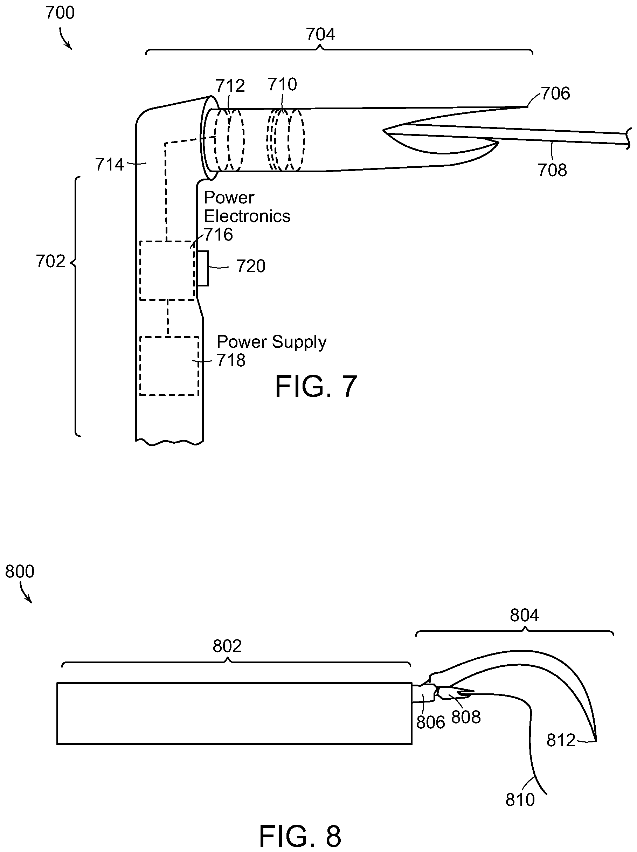

Insertion portion 404 includes an elongated base 406 that extends radially from handle portion 402 at a substantially perpendicular angle, in one embodiment, or in other embodiments any other angle may be utilized including acute or obtuse angles. Elongated base 406 may generally be of a cylindrical shape with a circular or ovoid diameter. In other embodiments, elongated base 406 may extend from handle portion 402 along a curved path, as shown in greater detail in FIG. 8. Elongated base 406 may be solid or may include a hollow portion that partially or fully extends along the length of elongated base 406. Any length can be selected for elongated base 406. Preferably, a minimal length for elongated base 406 is selected such that elongated base 406 is able to extend through the width of a layer of material while still allowing the greatest degree of movement for the operator.

At the end of insertion portion 404 opposite the junction between handle portion 402 and insertion portion 404 is a needle tip 408. Needle tip 408 is formed by tapering or beveling elongated base 406 in a direction away from handle portion 402 and is driven by elongated base 406 to pierce through a layer of material. The edge of such a bevel may be entirely blunt, entirely sharp, or only partially sharp.

Referring now to FIG. 5, the insertion portion 404 of stitching apparatus 400 is shown in greater detail. As shown, elongated base 406 may be of a hollow, cylindrical shape having an unbeveled portion 414 and a beveled portion 412 that exposes interior 416 of the hollow base 406. In some cases, the length and location of beveled portion 412 along elongated base 406 may be selected to increase the user's vision of interior 416 of insertion portion 404. For example, beveled portion 412 may extend along a greater length of elongated base 406 than that of unbeveled portion 414. In one embodiment, unbeveled portion 414 has a length that corresponds to the width of the tissue through which a thread is to extend as part of a stitch. The diameter of interior 416 can be selected to be slightly greater than the diameter of a thread tip affixed to the end of a thread such that the thread tip may be partially or fully inserted into interior 416 of elongated base 406.

In various embodiments, insertion portion 404 includes a thread extractor that engages a thread tip after insertion portion 404 has been driven through a layer of material. The thread extractor may be located within the hollow interior 406. In configurations in which elongated base 406 has a curved shape, the thread extractor may be located at any along the peripheral of elongated base 406 such that the thread extractor may engage the thread tip via a scooping action. The thread extractor may be located within unbeveled portion 414 along interior 416 and/or may extend from elongated base 406 into handle portion 402.

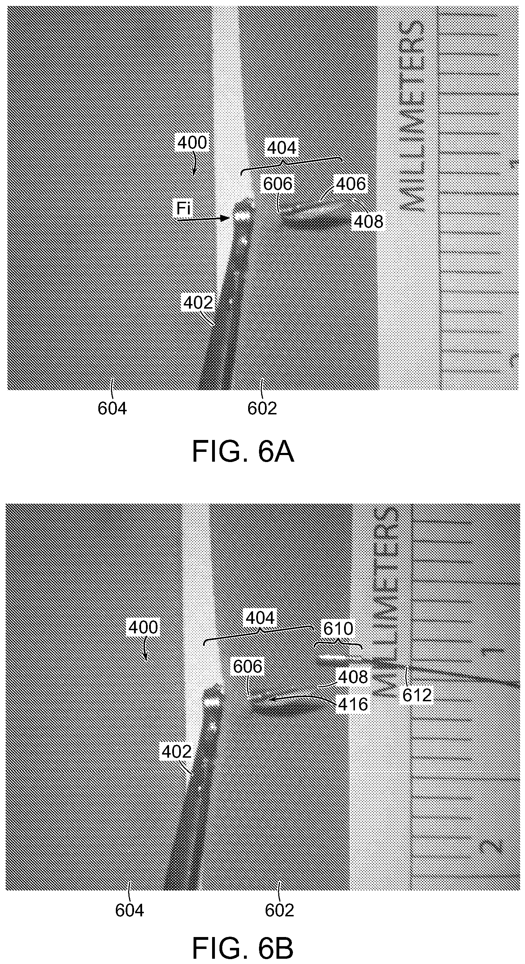

Referring now generally to FIGS. 6A-6D, the operation of stitching apparatus 400 is shown threading a piece of thread through a layer of material. As shown, one or more stitches can be applied between two layers of material 602, 604 using stitching apparatus 400, to bind materials 602, 604 together. Materials 602, 604 may be, but are not limited to, fabrics, polymers, combinations thereof, or the like. Thus, stitching apparatus can be adapted for use with any number of different materials having varying thicknesses and durabilities.

In FIG. 6A, stitching apparatus 400 is manipulated by a user via handle portion 402 to drive at least a portion of insertion portion 404 through material 602 at puncture point 606. An insertion force (Fᵢ) applied by the user via handle portion 402 is transferred through elongated base 406 to needle tip 408. For example, the insertion force may be applied to needle tip 408 along an axis that extends through elongated base 406 and needle tip 408 radially from handle portion 402. When the insertion force is applied to needle tip 408, needle tip pierces material 602 at puncture point 606. Additional application of the insertion force causes insertion portion 404 to be driven through the material 602.

In FIG. 6B, a thread 612 is moved within proximity of insertion portion 406 of stitching apparatus 400. Thread 612 may be any form of thread (e.g., wire, fiber, etc.) suitable for binding materials 602, 604 together. In surgical applications, thread 612 may be a suture thread designed for use with organic tissue and may be composed of an absorbable or non-absorbable material. For example, thread 612 may be composed of an absorbable material such as catgut, polyglycolic acid, polyactic acid, polydioxanone, caprolactone, or the like. Exemplary non-absorbable materials include polypropylene, polyester, nylon, metallic wires, and the like. In some cases, thread 612 may be coated with a compound that reduces friction during the stitching process, has antibacterial properties, and/or produces a biological reaction in the subject of the procedure (e.g., acts as an anti-inflammatory, etc.).

At one end of thread 610 suitably is a thread tip 610 that has a diameter equal to or greater than that of thread 610. As shown, thread tip 610 may be sized appropriately for insertion into interior 416 of elongated base 406, if elongated base 406 has a hollow configuration. In such a case, the bevel of elongated base 406 used to form needle tip 408 may be used to visually align thread tip 610 with interior 416 thread tip 610 to be inserted into elongated base 406. Thread tip 610 may be swaged or otherwise coupled to the end of thread 610 such that movement of thread tip 610 transfers a corresponding force to thread 610.

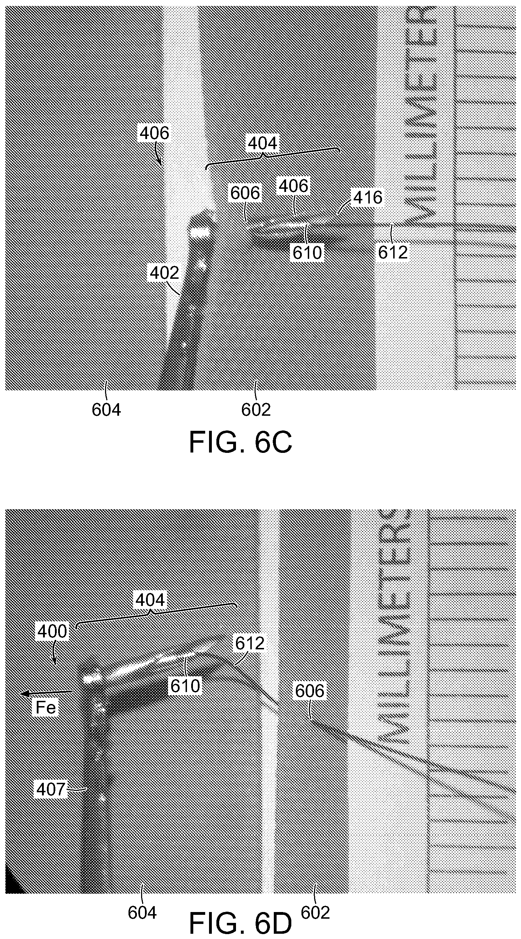

In FIG. 6C, thread tip 610 has been partially inserted into interior 416 of elongated base 406. The bevel of elongated base 406 acts to help retain thread tip 610 during insertion into stitching apparatus 400 and to provide the user with a visual indication of its progress. At full insertion into elongated base 406, thread tip 610 engages a thread extractor (not shown) that is located within interior 416 and couples thread tip 610 to stitching apparatus 400.

The internal thread extractor of stitching apparatus 400 may be of various configurations that permanently or releasably couple thread tip 610 to stitching apparatus 400. In one embodiment, the internal thread extractor may be a magnet and thread tip 610 may be composed of an oppositely polarized magnet or other material that exhibits magnetic properties when in the presence of a magnetic field. Thus, the thread extractor may exert a magnetic force that magnetically couples thread tip 610 to the thread extractor. The magnet of the thread extractor may be a permanent magnet or an electromagnet that is selectably powered to generate a magnetic field, in various embodiments. Thus, thread tip 610 may be released from the electromagnet at a later time either by removing the power to the electromagnet (e.g., to removing the magnetic force applied to thread tip 610) or by reversing the polarity of the magnetic field (e.g., by creating a magnetic force in the opposite direction, thereby forcing thread tip 610 out of extended base 406).

In cases in which a magnetic force is used to couple thread tip 610 and stitching apparatus 400, extended base 406 and/or thread 612 may be formed using non-magnetic material to prevent misalignment of thread tip 610 during use. For example, extended base 406 may be constructed using ceramics, carbon fiber, or another such material that does not react to the magnetic field of the thread extractor and retains its rigidity enough to pierce through material 602. Similarly, thread 612 may be constructed using a non-magnetic material, such as nylon, to prevent thread 612 from accidentally being attracted to stitching apparatus 400.

In another embodiment, the thread extractor may be an adhesive material applied within interior 416 of elongated base 406. For example, interior 416 may be coated with a glue that adheres thread tip 610 to interior 416. In another example, both interior 416 and thread tip 610 may be coated with compounds that chemically react to form a bond (e.g., a polyurethane resin / polyester resin pair or the like).

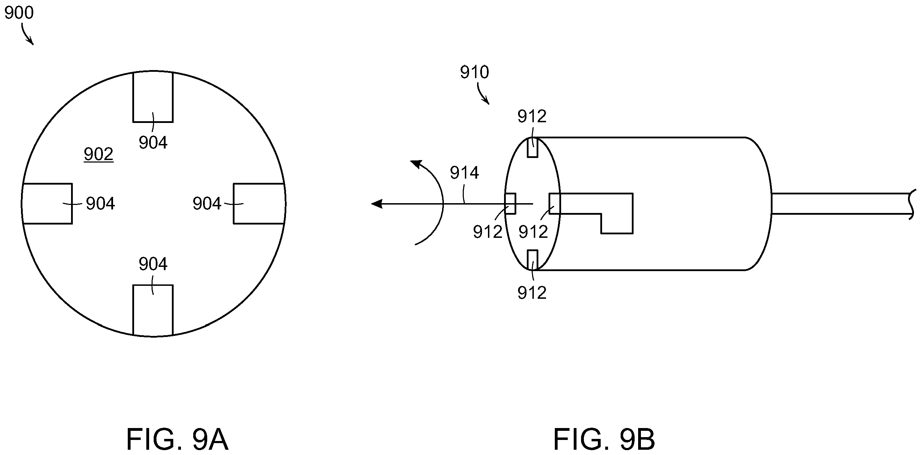

In yet further embodiments, the thread extractor may correspond to protrusions and/or apertures located within interior 416 of extended base 406. For example, hook and loop fasteners may be affixed to the exterior of thread tip 610 and to interior 416 of extended base 406 to engage thread tip 610 to stitching apparatus 400. In another example, one or more protrusions may be located within interior 416 that engage one or more slots located on thread tip 610 or vice-versa, as shown in more detail in FIGS. 9A-9B.

In FIG. 6D, an extraction force (Fₑ) is shown being applied to thread tip 610 to pull thread tip 610 and thread 612 through puncture point 606. The extraction force may be applied in substantially the opposite direction as the insertion force used to drive insertion portion 404 through material 602. During extraction, thread extractor transfers the extraction force exerted by the user on handle portion 402 to thread tip 610. Since the binding force exerted by thread extractor on thread tip 610 is greater than the extraction force, thread tip 610 is forced to move with insertion portion 404 back through puncture point 606, thereby extending thread 606 through material 602. After thread 612 has been threaded through puncture point 606, thread tip 610 may be decoupled from the thread extractor (e.g., by manually breaking the bond, by deactivating a magnetic bond, etc.). The steps depicted in FIGS. 6A-6D can then be repeated any number of times in an alternating fashion between materials 602, 604 to form stitches between the two using thread 606.

Notably, compared to traditional methods, there would be little resistance for thread to go through the puncture point 660, to another side of 602, since the puncture point 606 was pierced previously by the needle element, and the thread is now inside the hollow space 610'.

Referring now to FIG. 7, a schematic diagram of a stitching apparatus 700 is shown that uses an electromagnetic thread extractor. Similar to stitching apparatus 400, stitching apparatus 700 includes a handle portion 702 and an insertion portion 704 that includes a beveled needle tip 706. Insertion portion 704 defines a hollow space through which thread tip 710 has been inserted. Thread tip 710 is coupled to thread 708 such that movement of either thread tip 710 or thread 708 causes the other to move.

In various embodiments, insertion portion 704 includes an electromagnet 712 that exerts a magnetic force on thread tip 710 when active. The polarity of electromagnet 712 may be selected such that, when current is supplied to electromagnet 712, the resulting magnetic field draws thread tip 710 towards electromagnet 712 and couples thread tip 710 to electromagnet 712. In one embodiment, electromagnet 712 is a solenoid having windings along the interior of insertion portion 704 and extending radially away from handle portion 702. In another embodiment, electromagnet 712 is located partially or fully outside of the interior of insertion portion 704. In such a case, electromagnet 712 may be configured such that the flux path of its resulting magnetic field passes through at least a portion of the interior of insertion portion 704, thereby exerting a magnetic force on thread tip 710.

Stitching apparatus 700 may include power electronics 716 that provide current to the windings of electromagnet 712 from a power supply 718 via wires 714, to induce a magnetic field. Power electronics 716 and/or power supply 718 may be housed within handle portion. For example, power supply 718 may include one or more batteries (e.g., storage cells, supercapacitors, etc.) that allow stitching apparatus 700 to be fully portable. In another example, power supply 718 may be external to stitching apparatus 700 (e.g., power supply 718 may be an external battery, a wall socket, etc. coupled to power electronics 716).

Power electronics 716 operate to control the flow of current to electromagnet 712 in one or more directions. For example, power electronics 716 may have two or three modes of operation. In a dual mode configuration, power electronics 716 may induce an attractive magnetic field when active (e.g., forcing thread tip 710 toward stitching apparatus 400) and remove the magnetic field when deactivated. In a tri-mode configuration, power electronics 716 may induce an attractive magnetic field, induce a repulsive magnetic field by reversing the polarity to electromagnet 712 (e.g., forcing thread tip 710 away from stitching apparatus 400), or remove the current to electromagnet 712 completely to deactivate the magnetic field. In one embodiment, power electronics 716 includes a switch 720 located on the exterior or handle portion 702 that controls the mode of operation of power electronics 716.

Referring now to FIG. 8, a stitching apparatus 800 is shown, according to an exemplary embodiment. Stitching apparatus 800 includes a handle portion 802 that is grasped by a user to manipulate stitching apparatus 800. Stitching apparatus 800 also includes a curved insertion portion 804 connected to handle portion 802. At the end of insertion portion 804 opposite handle portion 802 is a needle tip 812 used to puncture through a layer of material in response to the user applying a force to handle portion 802. Such an insertion force may be at least semicircular in direction, allowing the user to "hook" insertion portion 804 through the layer of material.

As discussed above, a variety of other systems may be used to engage thread element and needle elements, including a vacuum system, for example where the needle element would exert a vacuum force to attract and engage the thread portion to the needle element.

At the base of insertion portion 804 is a thread extractor 806 that engages a thread tip 808 connected to thread 810. Thread extractor 806 may extend outward from handle portion 802 or may be located at least a fraction of the way into the interior of handle portion 802. In one embodiment, thread extractor 806 includes a magnet that attracts thread tip 808, thereby coupling thread tip 808 to stitching apparatus 800. In some cases, insertion portion 804 and/or handle portion 802 may define a hollow space that shrouds thread tip 808 when coupled to thread extractor 806. When thread tip 808 is coupled to thread extractor 806, an extraction force may be transferred from handle portion 802 to thread tip 808 to pull thread 810 through the puncture point created by needle tip 812 in the layer of material.

Referring now to FIGS. 9A-9B, cross sectional views of an insertion portion 900 of a stitching apparatus and a thread tip 910 are shown, respectively. In FIG. 9A, insertion portion 900 defines a hollow space 902 having a diameter slightly larger than that of thread tip 910, allowing thread tip 910 to be inserted into insertion portion 900. Protruding radially inward from the periphery of hollow space 902 is a plurality of locking protrusions 904. One or more locking protrusions 904 may be used, in various embodiments.

Thread tip 910 may include any number of apertures 912 that correspond to protrusions 904 of insertion portion 900. Apertures 912 may extend radially inward from the perimeter of thread tip 910 relative to an axis 914 that passes through the center of thread tip 910. Apertures 912 may be shaped such that two or more motions of insertion portion 900 and/or thread tip 910 causes protrusions 904 to engage apertures 912, thereby locking thread tip 910 into insertion portion 900. In some embodiments, apertures 912 of thread tip 910 may be of an "L" or "J" shape such that a first motion is used to insert protrusions 904 into apertures 912 and a second or subsequent motion is used to lock protrusions 904 into place. For example, thread tip 910 may be inserted into hollow space 902 of insertion portion 900 along axis 914, to first engage protrusions 904 with apertures 912. Thread tip 912 and/or insertion portion 900 may then be rotated about axis 914 to lock protrusions 904 into place on thread tip 910. Once locked into place, protrusions 904 transfer any subsequent force exerted to thread tip 910 along axis 914, such as an extraction force used to pull thread tip 910 through a layer of material.

In an alternate embodiment, apertures 912 may be located within hollow space 902 of insertion portion 900 and protrusions 904 may extend radially outward from thread tip 910. In yet another embodiment, apertures 912 and corresponding protrusions 904 may be distributed among thread tip 910 and insertion portion 900 such that each has at least one aperture and at least one protrusion.

The invention has been described in detail with reference to preferred embodiments thereof. However, it will be appreciated by those skilled in the art that changes may be made in these embodiments without departing from the principles of the invention, the scope of which is defined in the appended claims and their equivalents.

## Claims

1. A method of stitching a material (602, 604) different from living tissue, such as fabrics or polymers, comprising:
piercing a needle element (404, 704, 804, 900) through the material (602, 604);
engaging the needle element (404, 704, 804, 900) with a thread element (612, 708, 810) while the needle element (404, 704, 804, 900) extends through the material (602, 604);
withdrawing the needle element (404, 704, 804, 900) with the engaged thread element through the material (602, 604);
wherein the needle element (404, 704, 804, 900) comprises: a thread extractor which comprises a magnet that engages an end (610, 710, 808) of the thread element (612, 708, 810), which is composed of a material which exhibits magnetic properties when in presence of a magnetic field, by magnetically coupling the end (610, 710, 808) to the thread extractor;
a handle portion (402,702,802);
an insertion portion (404) that includes an elongated base (406) extending radially from the handle portion at an angle and the elongated base being hollow with the thread extractor being located within the hollow interior, and
a needle tip (408) formed on the elongated base by tapering or beveling the elongated base (406) in a direction away from the handle portion.

2. The method of claim 1, wherein the needle element (404, 704, 804, 900) releasably affixes to the thread element (612, 708, 810) during the engaging.

3. The method of claim 1 or 2, wherein the needle element (404, 900) mechanically engages the thread element (612).

4. The method of claim 1, 2 or 3, wherein the needle element (404) also engages the thread element (612) by an adhesive.

5. A stitching apparatus comprising:
a thread element (612, 708, 810), a needle element which comprises a thread extractor which comprises a magnet that engages an end (610, 710, 808) of the thread element (612, 708, 810), which is composed of a material which exhibits magnetic properties when in presence of a magnetic field, by magnetically coupling the end (610, 710, 808) to the thread extractor;
a handle portion (402,702,802);
an insertion portion that includes an elongated base extending radially from the handle portion at an angle and the elongated base being hollow with the thread extractor being located within the hollow interior, and
a needle tip formed on the elongated base by tapering or beveling the elongated base (406) in a direction away from the handle portion, and
means for transferring an insertion force from the handle portion (402, 702, 802) to the needle tip (408, 706, 812) in order to pierce through a layer of material (602, 604) using the needle tip (408, 706, 812) to insert the insertion portion (404, 704, 804, 900) through the layer of material (602, 604).

6. A needle system comprising the stitching apparatus of claim 5, wherein the thread element (612, 708, 810) is a suture element (612, 708, 810); and wherein the needle element (404, 704, 804, 900) and suture element (612, 708, 810) are configured to be releasably engaged together.

7. The needle system of claim 6, wherein an end portion of the needle element (404, 704, 804, 900) comprises an enclosure that houses the thread extractor and at least partially encases the tip (610, 710, 808, 910) of the suture element (612, 708, 810) when the suture element (612, 708, 810) is engaged to the thread extractor.

8. The needle system of claim 6 or 7, wherein the needle element (404, 704, 804) and suture element (612, 708, 810) each comprise a magnet.

9. The needle system of any one of claims 6 to 8 wherein the needle element (404, 704, 804) and suture element (612, 708, 810) each comprise mating mechanical engagements.

## Patentansprüche

1. Verfahren zum Nähen eines Materials (602, 604), das sich von lebendem Gewebe unterscheidet, wie Stoffgewebe oder Polymere, umfassend:
Durchstechen eines Nadelelements (404, 704, 804, 900) durch das Material (602, 604);
Ineingriffbringen des Nadelelements (404, 704, 804, 900) mit einem Fadenelement (612, 708, 810), während sich das Nadelelement (404, 704, 804, 900) durch das Material (602, 604) erstreckt;
Zurückziehen des Nadelelements (404, 704, 804, 900) mit dem in Eingriff stehenden Fadenelement durch das Material (602, 604);
wobei das Nadelelement (404, 704, 804, 900) umfasst: einen Fadenzieher, der einen Magneten umfasst, der an einem Ende (610, 710, 808) des Fadenelements (612, 708, 810) angreift, das aus einem Material besteht, das bei Vorhandensein eines Magnetfelds magnetische Eigenschaften aufzeigt, durch magnetisches Koppeln des Endes (610, 710, 808) mit dem Fadenzieher;
einen Griffabschnitt (402, 702, 802);
einen Einführabschnitt (404), der eine langgestreckte Basis (406) beinhaltet, die sich radial von dem Griffabschnitt in einem Winkel erstreckt und wobei die langgestreckte Basis hohl ist, wobei der Fadenzieher innerhalb des hohlen Innenraums angeordnet ist, und
eine Nadelspitze (408), die an der langgestreckten Basis durch Verjüngung oder Abschrägung der langgestreckten Basis (406) in einer Richtung vom Griffabschnitt weg gebildet wird.

2. Verfahren nach Anspruch 1, wobei das Nadelelement (404, 704, 804, 900) während des Ineingriffbringens lösbar an dem Fadenelement (612, 708, 810) befestigt ist.

3. Verfahren nach Anspruch 1 oder 2, wobei das Nadelelement (404, 900) mechanisch mit dem Fadenelement (612) in Eingriff gebracht wird.

4. Verfahren nach Anspruch 1, 2 oder 3, wobei das Nadelelement (404) auch durch einen Klebstoff mit dem Fadenelement (612) in Eingriff gebracht wird.

5. Nähvorrichtung, umfassend:
ein Fadenelement (612, 708, 810), ein Nadelelement, das umfasst:
einen Fadenzieher, der einen Magneten umfasst, der an einem Ende (610, 710, 808) des Fadenelements (612, 708, 810) angreift, das aus einem Material besteht, das bei Vorhandensein eines Magnetfelds magnetische Eigenschaften aufzeigt, durch magnetisches Koppeln des Endes (610, 710, 808) mit dem Fadenzieher;
einen Griffabschnitt (402, 702, 802);
einem Einführabschnitt, der eine langgestreckte Basis beinhaltet, die sich radial von dem Griffabschnitt in einem Winkel erstreckt und wobei die langgestreckte Basis hohl ist, wobei der Fadenzieher innerhalb des hohlen Innenraums angeordnet ist, und
eine Nadelspitze, die an der langgestreckten Basis durch Verjüngung oder Abschrägung der langgestreckten Basis (406) in einer Richtung vom Griffabschnitt weg gebildet ist, und Mittel zum Übertragen einer Einführkraft von dem Griffabschnitt (402, 702, 802) auf die Nadelspitze (408, 706, 812), um eine Materialschicht (602, 604) unter Verwendung der Nadelspitze (408, 706, 812) zu durchstechen, um den Einführabschnitt (404, 704, 804, 900) durch die Materialschicht (602, 604) einzuführen.

6. Nadelsystem, umfassend die Nähvorrichtung nach Anspruch 5, wobei das Fadenelement (612, 708, 810) ein Nahtelement (612, 708, 810) ist; und wobei das Nadelelement (404, 704, 804, 900) und das Nahtelement (612, 708, 810) konfiguriert sind, um lösbar miteinander in Eingriff gebracht zu werden.

7. Nadelsystem nach Anspruch 6, wobei ein Endabschnitt des Nadelelements (404, 704, 804, 900) eine Umhüllung umfasst, die den Fadenzieher aufnimmt und mindestens teilweise die Spitze (610, 710, 808, 910) des Nahtelements (612, 708, 810) einschließt, wenn das Nahtelement (612, 708, 810) mit dem Fadenzieher in Eingriff gebracht ist.

8. Nadelsystem nach Anspruch 6 oder 7, wobei das Nadelelement (404, 704, 804) und das Nahtelement (612, 708, 810) jeweils einen Magneten umfassen.

9. Nadelsystem nach einem der Ansprüche 6 bis 8, wobei das Nadelelement (404, 704, 804) und das Nahtelement (612, 708, 810) jeweils zusammenpassende mechanische Eingriffe umfassen.

## Revendications

1. Procédé de suture d'un matériau (602, 604) différent d'un tissu vivant, tel que des tissus ou des polymères, comprenant :
le perçage d'un élément d'aiguille (404, 704, 804, 900) à travers le matériau (602, 604) ;
la mise en prise de l'élément d'aiguille (404, 704, 804, 900) avec un élément de fil (612, 708, 810) pendant que l'élément d'aiguille (404, 704, 804, 900) s'étend à travers le matériau (602, 604) ;
le retrait de l'élément aiguille (404, 704, 804, 900) avec l'élément de fil en prise à travers le matériau (602, 604) ;
dans lequel l'élément d'aiguille (404, 704, 804, 900) comprend : un extracteur de fil qui comprend un aimant qui met en prise une extrémité (610, 710, 808) de l'élément de fil (612, 708, 810), qui est composé d'un matériau présentant des propriétés magnétiques en présence d'un champ magnétique, en couplant magnétiquement l'extrémité (610, 710, 808) à l'extracteur de fil ;
une partie de poignée (402, 702, 802) ;
une partie d'insertion (404) qui comporte une base allongée (406) s'étendant radialement à partir de la partie de poignée selon un angle et la base allongée étant creuse, l'extracteur de fil étant situé dans l'intérieur creux, et
une pointe d'aiguille (408) formée sur la base allongée en effilant ou en biseautant la base allongée (406) dans une direction s'éloignant de la partie de poignée.

2. Procédé selon la revendication 1, dans lequel l'élément d'aiguille (404, 704, 804, 900) se fixe de manière amovible à l'élément de fil (612, 708, 810) pendant la mise en prise.

3. Procédé selon la revendication 1 ou 2, dans lequel l'élément d'aiguille (404, 900) vient mécaniquement en prise avec l'élément de fil (612).

4. Procédé selon la revendication 1, 2 ou 3, dans lequel l'élément d'aiguille (404) vient également en prise avec l'élément de fil (612) par un adhésif.

5. Appareil de suture comprenant :
un élément de fil (612, 708, 810),
un élément d'aiguille qui comprend un extracteur de fil qui comprend un aimant qui met en prise une extrémité (610, 710, 808) de l'élément de fil (612, 708, 810), qui est composé matériau présentant des propriétés magnétiques en présence d'un champ magnétique, en couplant magnétiquement l'extrémité (610, 710, 808) à l'extracteur de fil;
une partie de poignée (402, 702, 802) ;
une partie d'insertion qui comporte une base allongée s'étendant radialement à partir de la partie de poignée selon un angle et la base allongée étant creuse, l'extracteur de fil étant situé dans l'intérieur creux, et
une pointe d'aiguille formée sur la base allongée en effilant ou en biseautant la base allongée (406) dans une direction s'éloignant de la partie de poignée, et
des moyens pour transférer une force d'insertion de la partie de poignée (402, 702, 802) à la pointe d'aiguille (408, 706, 812) afin de percer une couche de matériau (602, 604) en utilisant la pointe d'aiguille (408, 706, 812) pour insérer la partie d'insertion (404, 704, 804, 900) à travers la couche de matériau (602, 604).

6. Système d'aiguille comprenant l'appareil de suture selon la revendication 5, dans lequel l'élément de fil (612, 708, 810) est un élément de suture (612, 708, 810) ; et dans lequel l'élément aiguille (404, 704, 804, 900) et l'élément de suture (612, 708, 810) sont configurés pour être mis en prise ensemble de manière amovible.

7. Système d'aiguille selon la revendication 6, dans lequel une partie d'extrémité de l'élément d'aiguille (404, 704, 804, 900) comprend une enceinte qui loge l'extracteur de fil et enferme au moins partiellement la pointe (610, 710, 808, 910) de l'élément de suture (612, 708, 810) lorsque l'élément de suture (612, 708, 810) est mis en prise avec l'extracteur de fil.

8. Système d'aiguille selon la revendication 6 ou 7, dans lequel l'élément d'aiguille (404, 704, 804) et l'élément de suture (612, 708, 810) comprennent chacun un aimant.

9. Système d'aiguille selon l'une quelconque des revendications 6 à 8, dans lequel l'élément d'aiguille (404, 704, 804) et l'élément de suture (612, 708, 810) comprennent chacun des prises mécaniques d'accouplement.
